# EUROPÄISCHE PATENTSCHRIFT

(11) **EP 1 406 686 B1**
(45) Veröffentlichungstag und Bekanntmachung des Hinweises auf die Patenterteilung: **02.11.2005**
(21) Anmeldenummer: 02758345.9
(22) Anmeldetag: 15.07.2002
(51) Int. Cl.: A61M 5/28, A61M 5/32

(54) **VORRICHTUNG ZUR ABGABE VON MEDIEN**
DEVICE FOR DISTRIBUTING SUBSTANCES
DISPOSITIF POUR DELIVRER DES SUBSTANCES

(30) Priorität: 17.07.2001 DE 10134609
(43) Veröffentlichungstag der Anmeldung: 14.04.2004
(73) Patentinhaber: Hansen, Bernd, Dipl.-Ing., D-74429 Sulzbach-Laufen (DE)
(72) Erfinder: Hansen, Bernd, Dipl.-Ing., D-74429 Sulzbach-Laufen (DE)
(74) Vertreter: Bartels, Martin Erich Arthur
(86) Internationale Anmeldenummer: PCT/EP2002/007850
(87) Internationale Veröffentlichungsnummer: WO 2003/008021

(56) Entgegenhaltungen:
- WO-A-99/51290
- US-A- 4 139 009
- US-A- 4 735 618
- US-A- 5 836 922

## Beschreibung

Die Erfindung betrifft eine Vorrichtung zur Abgabe von Medien. Ein hauptsächlicher, jedoch nicht ausschließlicher, Verwendungszweck der Vorrichtung besteht darin, ein gewünschtes Volumen des Mediums, insbesondere eines flüssigen Mediums, in einen Aufnahmebehälter einzubringen. Vorzugsweise handelt es sich bei dem einzubringenden Medium um einen Zusatzstoff, der beispielsweise als Wirkstoffbeimengung in ein im Aufnahmebehälter befindliches Fluid eingebracht wird. Bei dem Aufnahmebehälter kann es sich um einen Infusionsbehälter handeln, zu dessen Inhalt das Medium als zusätzlicher Wirkstoff beizumengen ist.

Als Abgabevorrichtung wird hierzu üblicherweise eine Spritze benutzt, mit deren Kanüle ein perforierbarer Verschluß oder Stopfen des Aufnahmebehälters, beispielsweise des Infusionsbehälters, durchstochen wird, wonach durch Ausdrücken der Spritze das Medium in den Aufnahmebehälter injiziert wird. Bei diesem Vorgehen ist der vorbereitende Arbeitsschritt des Befüllens der Spritze erforderlich, indem die gewünschte Menge des Mediums aus einem Vorratsbehälter in die Spritze umgefüllt oder die Spritze aus einer konventionellen Phiole, die die abgemessene Dosis des betreffenden Mediums enthält, befüllt wird. Diese Arbeitsschritte des Umfüllens sind zum einen zeitraubend, weil Kanüle und Spritze ausgepackt, die Kanüle auf die Spritze montiert, die Phiole geöffnet oder angestochen werden und die Spritze aufgezogen werden müssen. Zum anderen besteht bei diesen Maßnahmen ein erhebliches Risiko der Kontamination des Mediums. Ein weiteres Problem bei derartigen Abgabevorrichtungen ist die durch die Kanülenspitze bedingte Verletzungsgefahr, der der Benutzer bei der Handhabung ausgesetzt ist. Im Hinblick hierauf ist bei einer bekannten Abgabevorrichtung, die in dem Dokument US 4,735,618 aufgezeigt ist, eine Sicherungseinrichtung vorgesehen, die einen das Kanülenende abdeckenden Sicherungskörper aufweist, der aus einem wirksamen Schutzzustand in einen für die Abgabe des Mediums aus der Kanüle vorgesehenen unwirksamen Gebrauchszustand überführbar ist. Zu diesem Zweck ist der Sicherungskörper entlang der Kanüle zwischen einer vorgeschobenen Schutzstellung am Ende der Kanüle und einer zurückgeschobenen Gebrauchsstellung verschiebbar.

Nachteilig ist bei dieser bekannten Vorrichtung, dass keine Vorkehrungen gegen ein unbeabsichtigtes Überführen in die Gebrauchsstellung gegeben sind, in der das Kanülenende frei vorsteht. Die bekannte Vorrichtung ist lediglich nach erfolgtem Gebrauch sicherbar, indem die Kanülenspitze in eine Aufnahmevertiefung des Sicherheitskörpers eingehängt wird, nachdem dieser nach dem Gebrauch der Vorrichtung über die Kanülenspitze hinaus vorgeschoben wird. Die bekannte Vorrichtung ist daher vom Sicherheitsstandpunkt her unbefriedigend.

Ausgehend von diesem Stand der Technik stellt sich die Erfindung die Aufgabe, eine Vorrichtung zu schaffen, die eine erhöhte Sicherheit gegen Verletzungen bei Transport, Lagerung und Gebrauch bietet.

Erfindungsgemäß ist diese Aufgabe durch eine Abgabevorrichtung gelöst, die die Merkmale des Patentanspruches 1 aufweist.

Dadurch, dass erfindungsgemäß eine das Ende der Kanüle umschließende, vom Abgabebehälter abnehmbare Schutzhaube vorgesehen ist, ist nicht nur die Gefahr eines unbeabsichtigten Zurückschiebens des Sicherungskörpers der Sicherungseinrichtung vermieden, sondern es ist zusätzlich auch eine erhöhte Sicherheit gegen Kontamination erreicht. Dank des Vorhandenseins eines an der Schutzhaube befindlichen Drehknebels zum Abdrehen der Schutzhaube vom Abgabebehälter, mit dem die Schutzhaube über eine Sollbruchstelle verbunden ist, gestaltet sich auch die Handhabung der erfindungsgemäßen Vorrichtung besonders einfach und bequem.

Wenn das äußere Ende der Kanüle zum Durchstoßen eines perforierbaren Verschlusses eines das abzugebende Medium aufnehmenden Behälters vorgesehen ist, kann der Sicherungskörper beim Durchstoßen des Verschlusses durch Anlage an demselben aus seiner vorgeschobenen Schutzstellung am Ende der Kanüle in die Gebrauchsstellung zurückgeschoben und nach Herausziehen der Kanüle aus dem Verschluß wieder in die Schutzstellung vorgeschoben werden. Bei einem derartigen Ausführungsbeispiel ist die Handhabung besonders vereinfacht.

Als Sicherungskörper kann die Sicherungseinrichtung einen auf dem aus der Verschlußeinheit vorstehenden Abschnitt der Kanüle verschiebbar geführten Ringkörper aufweisen, der mit der Verschlußeinheit über eine eine Haltekraft erzeugende, nachgiebige Halteeinrichtung verbunden, durch deren Haltekraft in der das Ende der Kanüle abdeckenden, vorgeschobenen Schutzstellung kraftschlüssig gehalten und gegen die Haltekraft entlang der Kanüle in die Gebrauchsstellung zurückschiebbar ist. Der genannte Ringkörper bildet an seiner freien Stirnseite eine ringförmige, verbreiterte Anlagefläche aus, die an der vorderen Stelle der Sicherungseinrichtung radial über deren sonstige Teile vorsteht und über die verbreiterte Anlagefläche ergibt sich ein sicheres Anlageverhalten des Ringkörpers mit der durchstoßbaren Stelle des Behälters an seiner Entnahmeöffnung. Ferner kann aufgrund der Anlagefläche beim Durchstoßen des perforierbaren Verschlusses des Abgabebehälters die Kanüle nicht verkanten oder an der empfindlichen Spitze beschädigt werden.

Die Halteeinrichtung kann durch mittels Gelenken unterteilte, an Ringkörper und Verschlußeinheit jeweils gelenkig angebrachte Trägerelemente gebildet sein, die sich bei der Schutzstellung im wesentlichen parallel zur Kanüle erstrecken und, wenn der Ringkörper längs der Kanüle in die Gebrauchsstellung zurückgeschoben wird, sich in von der Kanüle abgespreizter Lage erstrecken.

Vorzugsweise sind die Trägerelemente einstückig an die Verschlußeinheit angeformte, stabartige Kunststoffteile, die auf etwa der halben Länge durch Biegegelenke unterteilt sind. Bei solcher Ausbildung ermöglicht die Eigenelastizität des Werkstoffes der Trägerelemente, daß sich der Ringkörper nach dem Zurückschieben in die Gebrauchsstellung selbsttätig wieder zumindest teilweise gegen das vordere Ende der Kanüle vorschiebt, daß der Ringkörper also selbsttätig wieder in die Schutzstellung zurückkehrt oder nach dem Gebrauch der Vorrichtung lediglich geringfügig weiter nach vorn vorgeschoben werden muß. Da die Biegegelenke seitlich vollständig abklappen können, können die ein jeweiliges Biegegelenk bildenden, stabartigen Kunststoffteile in Anlage übereinandergelegt werden, so daß eine hohe Eindringtiefe der Kanüle in den Abgabebehälter erreichbar ist und auch perforierbare Verschlüsse mit entsprechenden Dickenabmessungen des Abgabebehälters lassen sich dergestalt gezielt mittels der Kanüle durchstoßen.

Bei einem besonders vorteilhaften Ausführungsbeispiel trägt die Verschlußeinheit einen die Trägerelemente umgebenden Sicherungsring, der von der Verschlußeinheit abnehmbar und entlang der Trägerelemente zu deren Biegegelenken hin verschiebbar ist, um diese in der der Schutzstellung entsprechenden Stellung zu blockieren. Hierbei können an den Biegegelenken Rastkerben vorgesehen sein, in die der Sicherungsring einschnappen kann, so daß die Sicherungseinrichtung in der Schutzstellung lösbar verriegelt wird, in der der Ringkörper das äußere Ende der Kanüle schützend umgibt.

Der Abgabebehälter kann ein nach dem bekannten Bottelpack® - Verfahren hergestellter Kunststoffbehälter sein, in dessen offenes Ende vor dem Verschließen mittels der Schutzhaube die Verschlußeinheit als Einlegeteil eingelegt ist.

Der Körper des Einlegeteils enthält im Zentralbereich die in ihm durchgehend angeordnete Kanüle, deren inneres Ende aus dem Körper der Verschlußeinheit gegen den Innenraum des Abgabebehälters hin geringfügig vorsteht. Das die Verschlußeinheit bildende Einlegeteil weist zwischen dem zugekehrten inneren Ende der Kanüle und dem im Abgabebehälter befindlichen Medium eine Membran als Sperrelement auf, die, wenn der Druck des im Abgabebehälter befindlichen Mediums erhöht wird, gegen das Kanülenende gedrückt und von diesem durchstoßen wird.

Das Erhöhen des Druckes des Mediums und das dadurch erfolgende Durchstoßen der Membran kann bewirkt werden, indem der Abgabebehälter zusammengedrückt wird.

Nachstehend ist die Erfindung anhand eines in der Zeichnung dargestellten Ausführungsbeispiels im einzelnen erläutert.
Es zeigen:
- Fig.1: eine Seitenansicht eines Ausführungsbeispiels der erfindungsgemäßen Abgabevorrichtung in dem Betriebszustand vor dem Gebrauch, d.h. mit aufgesetzter Schutzhaube;
- Fig.2: einen Längsschnitt der Vorrichtung von Fig.1;
- Fig.3: einen der Fig.2 entsprechenden Längsschnitt, jedoch mit abgenommener Schutzhaube;
- Fig.4: einen Längsschnitt der Vorrichtung in dem in einen perforierbaren Verschlußstopfen eines Infusionsbehälters eingestochenen Betriebszustand;
- Fig.5: eine der Fig.4 ähnliche Darstellung, wobei jedoch der Abgabebehälter der Vorrichtung ausgedrückt ist;
- Fig.6: einen Längsschnitt der Vorrichtung in ausgedrücktem und aus dem Verschlußstopfen abgezogenen Zustand und
- Fig.7: einen Längsschnitt der Vorrichtung in dem ausgedrückten, der Schutzstellung der Sicherungseinrichtung entsprechenden Betriebszustand.

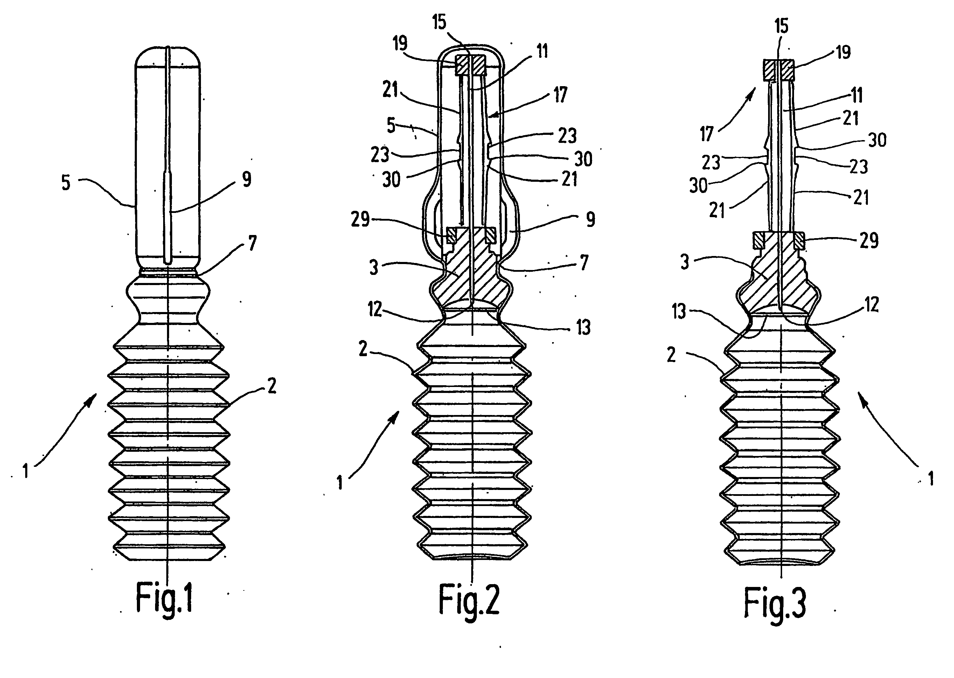

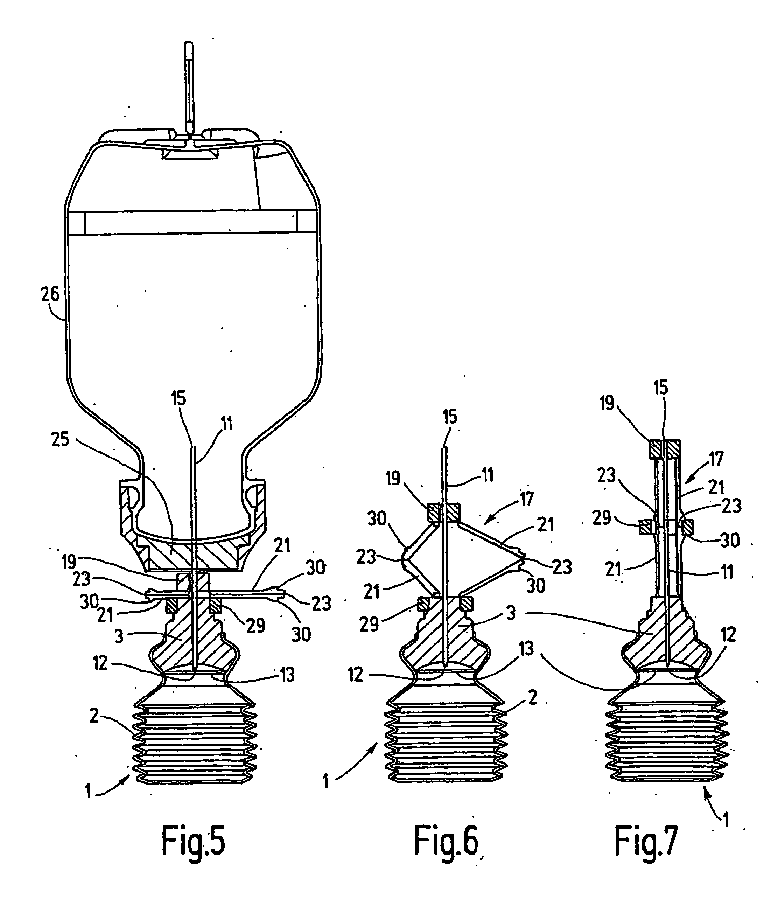

In den Figuren ist ein Abgabebehälter als Ganzes mit 1 bezeichnet. Dieser ist beim vorliegenden Ausführungsbeispiel ein nach dem bekannten Bottelpack®-Verfahren hergestellter, ampullenartiger Kunststoffbehälter, dessen Wand 2 mit Falten versehen und balgartig gestaltet ist, so daß der Abgabebehälter 1 aus der in Fig. 1 bis 4 gezeigten Konfiguration zusammengedrückt werden kann, siehe Fig.5 bis 7. Im Halsbereich ist in den Abgabebehälter 1 als Einlegeteil eine Verschlußeinheit 3 eingesetzt, an deren aus Kunststoff geformten Körper mehrere Bauteile angeformt sind. Unter anderem ist dies eine Sicherungseinrichtung 17, welche ihrerseits (vor dem Gebrauch der Abgabevorrichtung) geschützt ist durch eine Schutzhaube 5, die an einer Sollbruchstelle 7, siehe Fig.1 und 3, an den Körper des Abgabebehälters 1 angeformt ist und einen seitlich auskragenden Drehknebel 9 aufweist, der als Handhabe dient, um die Schutzhaube 5 an der Sollbruchstelle 7 vom Abgabebehälter 1 abzudrehen. Fig.3 zeigt den gleichen Betriebszustand der Vorrichtung wie Fig.1 und 2, jedoch mit abgenommener Schutzhaube 5.

Wie insbesondere aus Fig. 2 und 3 zu ersehen ist, ist der Körper der Verschlußeinheit 3 im Zentralbereich von einer Kanüle 11 durchzogen, deren inneres Ende 12 über den Körper der Verschlußeinheit 3 nach innen leicht vorsteht. Zwischen dem inneren Ende 12 der Kanüle 11 und dem Innenraum des Abgabebehälters 1 befindet sich eine Membran 13, die Teil des Einlegeteils der Verschlußeinheit 3 ist.

Die Kanüle erstreckt sich vom äußeren Ende des Körpers der Verschlußeinheit 3 in einer Länge, die in etwa der Länge einer Spritzenkanüle entspricht. Die Fig.2, 3 und 7 zeigen Betriebszustände, bei denen das vorstehende äußere Ende 15 der Kanüle 11 jeweils durch eine in ihrer Schutzstellung befindliche Sicherungseinrichtung, die als Ganzes mit 17 bezeichnet ist, abgedeckt, d.h. geschützt ist.

Die Sicherungseinrichtung 17 ist an den Körper der Verschlußeinheit 3 einstückig angeformt und weist einen Ringkörper 19 auf, der auf der Kanüle 11 verschiebbar ist und sich in der Schutzstellung, siehe Fig.2, 3 und 7, am äußeren Ende 15 der Kanüle 11 befindet, um dieses Kanülenende, d.h. die Nadelspitze, abzudecken. Der Ringkörper 19 ist über einstückig angeformte Trägerelemente 21 stabartiger Gestalt mit dem Körper der Verschlußeinheit 3 einstückig verbunden, wobei die Verbindungsstellen mit Ringkörper 19 und Körper der Verschlußeinheit 3 in der Art von Biegegelenken nachgiebig sind. Außerdem befinden sich auf etwa der halben Länge der Trägerelemente 21 Biegegelenke 23, die die Trägerelemente 21 unterteilen. Wird der Ringkörper 19 aus der Schutzstellung in die Gebrauchsstellung der Vorrichtung längs der Kanüle 11 verschoben, siehe Fig.5 und 6, so schwenken sich die an die Biegegelenke 23 angrenzenden Abschnitte der Trägerelemente 21, so daß sie sich von der Kanüle 11 abspreizen und so zusammenlegen, wie dies in Fig.5 gezeigt ist.

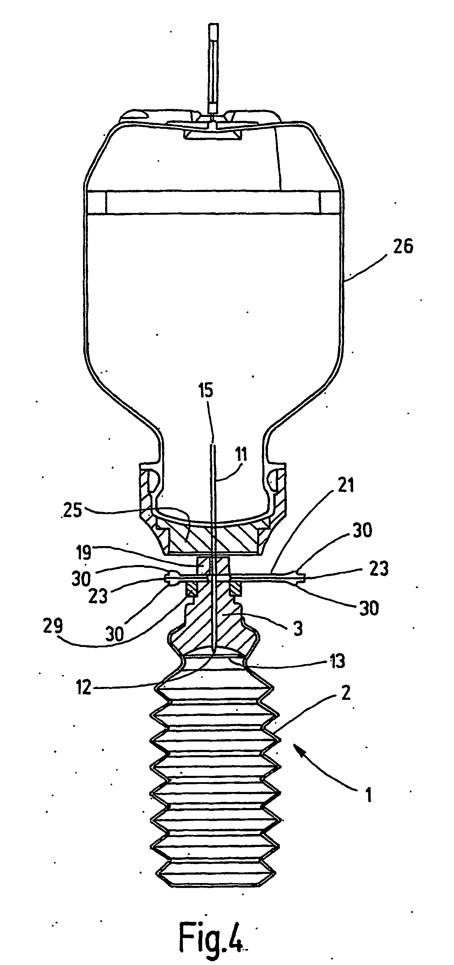

Die letztgenannte Fig.5 und Fig.4 zeigen die Vorrichtung in dem Zustand, wo die Kanüle 11 mit ihrem vorderen Ende 15 einen perforierbaren Verschluß 25 eines Infusionsbehälters 26 durchstochen hat. Hierbei ist der Ringkörper 19 aus der auf das Ende 15 der Kanüle ausgerichteten Schutzstellung längs der Kanüle 11 in die Gebrauchsstellung zurückgeschoben. Durch Zusammendrücken der balgartigen Wand 2 des Abgabebehälters 1 (siehe Fig.5) ist der Druck des darin befindlichen Mediums erhöht worden, so daß die Membran 13 gegen das zugewandte Ende 12 der Kanüle 11 gepreßt und von dieser durchstochen wird. Das Zusammendrücken des Abgabebehälters 1 führt dadurch zu einem Ausdrücken des darin befindlichen Mediums in den Infusionsbehälter 26 hinein, so daß zu dessen Inhalt eine dem Inhalt des Abgabebehälters 1 entsprechende Dosis eines Zusatz- oder Wirkstoffes beigemengt wird.

Fig.6 zeigt den Betriebszustand, nachdem die Vorrichtung mit dem ausgedrückten Abgabebehälter 1 wieder aus dem Verschluß 25 des Infusionsbehälters 26 abgezogen ist. Aufgrund der Eigenelastizität der Trägerelemente 21 hat sich der Ringkörper 19, welcher zuvor beim Einstechen der Kanüle in den Verschluß 25 durch Anlage an denselben gegen die Elastizitäts- oder Haltekraft der Trägerelemente 21 aus der Schutzstellung zurückgeschoben worden ist, selbsttätig durch die Elastizitätskraft wieder teilweise gegen das Ende 15 der Kanüle 11 hin vorgeschoben.

Fig.7 zeigt den Betriebszustand der Vorrichtung nach erfolgtem Gebrauch derselben, wobei das vorstehende äußere Ende 15 de Kanüle 11, obgleich die Schutzhaube 5 nicht mehr angebracht ist, durch die Sicherungseinrichtung 17 wieder gesichert ist. Zu diesem Zweck ist ein auf dem Körper der Verschlußeinheit 3 abnehmbar sitzender Sicherungsring 29 von der Verschlußeinheit abgenommen und längs der Kanüle 11 vorgeschoben, wobei er über die Trägerelemente 21 gleitet und diese aus der in Fig.6 gezeigten Position an die Kanüle 11 annähert, wobei der Ringkörper 19 bis zum Ende 15 der Kanüle 11 vorgeschoben wird. An den Biegegelenken 23 weisen die Trägerelemente 21 angeformte Rastkerben 30 auf, in die der Sicherungsring 29 einrastet, siehe Fig.7.

Nach dem Einrasten des Sicherungsringes 29 in die Rastkerben 30 an den Biegegelenken 23 der Trägerelemente 21 ist die Kanüle 11 durch den ihr Ende 15 abdeckenden Ringkörper 19 trotz abgenommener Schutzhaube 5 wiederum abgedeckt, so daß die nun entleerte Vorrichtung gefahrlos entsorgt werden kann. Es versteht sich, daß die erfindungsgemäße Abgabevorrichtung nicht nur zur Beimengung gewünschter Volumina flüssiger Medien in Infusionsbehälter mit Vorteil benutzt werden kann, sondern gleichermaßen für die Abgabe flüssiger oder gasförmiger und/oder partikelbefrachteter Medien, soweit deren Abgabe über Kanülen möglich oder erforderlich ist, angewendet werden kann.

## Patentansprüche

1. Vorrichtung zur Abgabe von Medien, mit einem das betreffende Medium aufnehmenden Abgabebehälter (1) mit einer Verschlußeinheit (3), die eine Kanüle (11) für das abzugebende Medium aufweist, deren äußeres Ende (15) aus der Verschlußeinheit (3) vorsteht, und mit einer Sicherungseinrichtung (5,17), die aus einem das äußere Ende (15) der Kanüle (11) abdeckenden wirksamen Schutzzustand in einen für die Abgabe des Mediums aus der Kanüle (11) vorgesehenen unwirksamen Gebrauchszustand überführbar ist und einen Sicherungskörper (19) aufweist, der aus einer vorgeschobenen Schutzstellung am Ende (15) der Kanüle (11) in eine Gebrauchsstellung zurückschiebbar und wieder in die Schutzstellung vorschiebbar ist, **dadurch gekennzeichnet, daß** die Sicherungseinrichtung (5, 17) eine das vorstehende Ende (15) der Kanüle (11) umschließende, vom Abgabebehälter (1) abnehmbare Schutzhaube (5) aufweist, die mit dem Abgabebehälter über eine Sollbruchstelle (7) verbunden ist, und daß die Schutzhaube (5) einen Drehknebel (9) als Handhabe zum Abdrehen der Schutzhaube (5) vom Abgabebehälter (1) aufweist.

2. Vorrichtung nach Anspruch 1, **dadurch gekennzeichnet, daß** das äußere Ende (15) der Kanüle (11) zum Durchstoßen eines perforierbaren Verschlusses (15) eines das abzugebende Medium aufnehmenden Behälters (26) vorgesehen ist und daß der Sicherungskörper (19) beim Durchstoßen des Verschlusses (25) durch Anlage an demselben aus seiner vorgeschobenen Schutzstellung in die Gebrauchsstellung zurückschiebbar ist.

3. Vorrichtung nach Anspruch 1 oder 2, **dadurch gekennzeichnet, daß** der Sicherungskörper (19) der Sicherungseinrichtung (5,17) durch einen auf dem aus der Verschlußeinheit (3) vorstehenden Abschnitt der Kanüle (11) verschiebbar geführten Ringkörper (19) gebildet ist, der mit der Verschlußeinheit (3) über eine eine Haltekraft erzeugende, nachgiebige Halteeinrichtung (21) verbunden, durch deren Haltekraft in der das Ende (15) der Kanüle (11) abdeckenden, vorgeschobenen Schutzstellung kraftschlüssig gehalten und gegen die Haltekraft entlang der Kanüle (11) in die Gebrauchsstellung zurückschiebbar ist.

4. Vorrichtung nach Anspruch 3, **dadurch gekennzeichnet, daß** die Halteeinrichtung durch Gelenke unterteilte, an Ringkörper (19) und Verschlußeinheit (3) jeweils gelenkig angebrachte Trägerelemente (21) aufweist, die sich bei der Schutzstellung im wesentlichen parallel zur Kanüle (11) und in der Gebrauchsstellung in von dieser abgespreizter Lage erstrecken.

5. Vorrichtung nach Anspruch 4, **dadurch gekennzeichnet, daß** die Trägerelemente (21) sich in einstückigem Verlauf von der Verschlußeinheit (3) zum Ringkörper (19) erstrecken und auf etwa halber Länge durch Biegegelenke (23) unterteilt sind.

6. Vorrichtung nach Anspruch 5, **dadurch gekennzeichnet, daß** die Verschlußeinheit (3) einen die Trägerelemente (21) umgebenden Sicherungsring (29) trägt, der von der Verschlußeinheit (3) abnehmbar und entlang der Trägerelemente (21) zu deren Biegegelenken (23) hin verschiebbar ist, um diese in der der Schutzstellung entsprechenden Stellung zu blockieren.

7. Vorrichtung nach einem der Ansprüche 1 bis 6, **dadurch gekennzeichnet, daß** die Verschlußeinheit (3) eine Membran (13) besitzt, die zwischen dem im Abgabebehälter (1) befindlichen Medium und dem zugekehrten inneren Ende (12) der Kanüle (11) ein Sperrelement bildet, das bei Erhöhen des Druckes des Mediums an das Ende (12) der Kanüle (11) andrückbar und durch diese durchstoßbar ist.

8. Vorrichtung nach Anspruch 7, **dadurch gekennzeichnet, daß** der Abgabebehälter (1) eine verformbare Wand (2) aufweist und für das das Durchstoßen der Membran (13) bewirkende Erhöhen des Druckes des Mediums zusammendrückbar ist.

9. Vorrichtung nach Anspruch 8, **dadurch gekennzeichnet, daß** die Wand (2) des Abgabebehälters (1) in der Art eines Faltenbalges ausgebildet ist.

## Claims

1. Device for dispensing of media, with a dispensing container (1) with a closure unit (3) receiving the relevant medium, incorporating a cannula (11) for the medium to be dispensed, the outermost end (15) of which projects from the closure unit (3), and with a safety means (5, 17) which can be transformed from an effective protective position in which it covers the outermost end (15) of the cannula (11) into an ineffective operating position envisaged for the dispensing of the medium from the cannula (11), and incorporating a safety body (19) which can be pushed back into an operating position and forwards into the protective position from a projecting protective position at the end (15) of the cannula (11), **characterised in that** the safety means (5, 17) incorporates a protective hood (5) enclosing the projecting end (15) of the cannula (11), which can be removed from the dispensing container (1), the same being connected with the dispensing container via a perforation (7), and **in that** the protective hood (5) incorporates a turning knob (9) as a handle for twisting off the protective hood (5) from the dispensing container (1).

2. Device according to Claim 1, **characterised in that** the outermost end (15) of the cannula (11) is envisaged for penetrating a perforatable closure (15) of a container (26) receiving the medium to be dispensed, and **in that** the safety body (19) can be pushed back during the penetration of the closure (25) by abutting it to the same from its projected protective position into the operating position.

3. Device according to Claim 1 or 2, **characterised in that** the safety body (19) of the safety means (5, 17) is formed by an annular body (19) displaceably held on the section of the cannula (11) that projects from the closure unit (3), whereby the same is connected with the closure unit (3) via a yielding holding means (21), which together create a holding force, said holding force positively connecting the same in the projected protective position in which the end (15) of the cannula (11) is covered and being retractable against said holding force along the cannula (11) into the operating position.

4. Device according to Claim 3, **characterised in that** the holding means incorporates supporting elements (21) sub-divided by joints moveably affixed to annular body (19) and closure means (3), the same extending substantially parallel with the cannula (11) in the protective position and away from the same in the operating position.

5. Device according to Claim 4, **characterised in that** the supporting elements (21) extend in the direction of the annular body (19) in one piece from the closure unit (3), and are sub-divided approximately at half length by means of bending elements (23).

6. Device according to Claim 5, **characterised in that** the closure unit (3) is equipped with a safety ring (29) surrounding the supporting elements (21), the same being removable by the closure unit (3) and displaceable along the supporting elements (21) in the direction of the bending elements (23) in order to block the same in the position that equals the protective position.

7. Device according to one of the Claims 1 to 6, **characterised in that** the closure unit (3) incorporates a membrane (13) which forms a barrier element between the medium inside the dispensing container (1) and the facing inner end (12) of the cannula (11), whereby the same can be pressed against the end of the cannula (11) when the pressure of the medium is increased and can perforate the same.

8. Device according to Claim 7, **characterised in that** the dispensing container (1) incorporates a deformable wall (2) for compressing the medium and increasing the pressure, therefore effecting the perforation of the membrane (13).

9. Device according to Claim 8, **characterised in that** the wall (2) of the dispensing container (1) takes the form of bellows.

## Revendications

1. Dispositif pour la distribution de fluides, comprenant :
- un récipient distributeur (1) qui recueille le fluide concerné et qui est muni d'une unité de fermeture (3) présentant une canule (11) pour le fluide à distribuer dont l'extrémité extérieure (15) dépasse de l'unité de fermeture (3) ; et
- un dispositif de sécurité (5, 17) qui peut être transformé depuis un état de protection efficace recouvrant l'extrémité extérieure (15) de la canule (11) jusque dans un état d'utilisation inefficace prévu pour la distribution du fluide depuis la canule (11), et qui présente un corps de sécurité (19) pouvant être repoussé en arrière dans un état d'utilisation depuis une position de protection avancée sur l'extrémité (15) de la canule (11) et pouvant être repoussé à nouveau en avant dans la position de protection,
**caractérisé en ce que** :
- le dispositif de sécurité (5, 17) présente une calotte de protection (5) qui entoure l'extrémité en saillie (15) de la canule (11), qui peut être retirée du récipient distributeur (1) et qui est reliée au réservoir distributeur via un point destiné à la rupture (7) ; et
- la calotte de protection (5) présente une manette rotative (9) comme ustensile pour fermer la calotte de protection (5) du récipient distributeur (1).

2. Dispositif selon la revendication 1, **caractérisé en ce que** l'extrémité extérieure (15) de la canule (11) est prévue pour percer une fermeture (15) d'un récipient qui recueille le fluide à distribuer, ladite fermeture pouvant être perforée, et **en ce que** le corps de sécurité (19) peut être repoussé en arrière depuis sa position de protection avancée jusque dans la position d'utilisation, lors de la percée de la fermeture (25) par contact sur celui-ci.

3. Dispositif selon la revendication 1 ou 2, **caractérisé en ce que** le corps de sécurité (19) du dispositif de sécurité (5, 17) est formé par un corps annulaire (19) qui est guidé en translation sur le tronçon de la canule (11) dépassant de l'unité de fermeture (3) et qui est relié à l'unité de fermeture (3) via un dispositif de retenue (21) générant une force de retenue et faisant ressort, la position de protection avancée qui recouvre l'extrémité (15) de la canule (11) étant maintenue par coopération de forces via la force de retenue dudit dispositif de retenue et pouvant être repoussée en arrière le long de la canule (11) jusque dans la position d'utilisation, à l'encontre de la force de retenue.

4. Dispositif selon la revendication 3, **caractérisé en ce que** le dispositif de retenue présente des éléments porteurs (21), subdivisés par des articulations et montés respectivement en articulation sur le corps annulaire (19) et sur l'unité de fermeture (3), lesquels s'étendent essentiellement parallèlement par rapport à la canule (11) dans la position de protection et à l'écart de celle-ci dans la position d'utilisation.

5. Dispositif selon la revendication 4, **caractérisé en ce que** les éléments porteurs (21) s'étendent en un seul tenant depuis l'unité de fermeture (3) vers le corps annulaire (19) et sont subdivisés par des articulations flexibles (23) approximativement à mi-longueur.

6. Dispositif selon la revendication 5, **caractérisé en ce que** l'unité de fermeture (3) porte un anneau de sécurité (29) qui entoure les éléments porteurs (21), qui peut être retiré de l'unité de fermeture (3) et qui peut être déplacé le long des éléments porteurs (21) vers leurs articulations flexibles (23) afin de bloquer ceux-ci dans la position correspondant à la position de protection.

7. Dispositif selon l'une des revendications 1 à 6, **caractérisé en ce que** l'unité de fermeture (3) possède une membrane (13) qui forme un élément barrière entre le fluide se trouvant dans le récipient distributeur (1) et l'extrémité intérieure adjacente (12) de la canule (11), lequel peut être pressé sur l'extrémité (12) de la canule (11) lors de l'augmentation de la pression et lequel peut être percé par celle-ci.

8. Dispositif selon la revendication 7, **caractérisé en ce que** le récipient distributeur (1) présente une paroi déformable (2) et peut être comprimé pour l'augmentation de la pression du fluide provoquant la percée de la membrane (13).

9. Dispositif selon la revendication 8, **caractérisé en ce que** la paroi (2) du récipient distributeur (1) est réalisée à la manière d'un soufflet.
